# EUROPEAN PATENT APPLICATION

(11) **EP 4 692 796 A1**
(43) Date of publication of application: **11.02.2026**
(21) Application number: 24780809.0
(22) Date of filing: 29.03.2024
(51) Int. Cl.: G01N 33/543

(54) **REAGENT, KIT, AND METHOD FOR DETECTING SUBSTANCE TO BE MEASURED**

(30) Priority: 30.03.2023 JP 2023055137
(71) Applicant: FUJIFILM Corporation, Tokyo 106-8620 (JP)
(72) Inventor: YAMASE Kenya, Tokyo 106-8620 (JP)
(74) Representative: Dehns Germany Partnerschaft mbB
(86) International application number: PCT/JP2024/013047
(87) International publication number: WO 2024/204714

(57) **Abstract**

An object of the present invention is to provide a reagent capable of reducing a non-specific reaction in the detection of a substance to be measured in a saliva sample using Substance 1 that is labeled with a peroxidase and that binds to the substance to be measured, a kit including the reagent, and a method for measuring a substance to be measured using the reagent. According to the present invention, there are provided a reagent containing a glycoprotein, in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured; a kit containing the reagent; and a method for measuring of a substance to be measured using the reagent.

## Description

### Technical Field

The present invention relates to a reagent, a kit, and a method for detecting a substance to be measured.

### Background Art

Currently, detection of a substance to be measured in a saliva sample is performed in various fields.

For example, in the measurement of a marker of an infectious disease such as that caused by severe acute respiratory syndrome coronavirus-2 (SARS-CoV-2) and the measurement of a hormone or an antibody in saliva, it is known that an immunological examination such as an enzyme-linked immunosorbent assay (ELISA) and a chemiluminescent enzyme immunoassay (CLEIA) is performed by collecting a saliva sample.

Here, the examination using the saliva sample has advantages such as easy collection, less invasiveness at the time of collection, and a low risk of contaminating the collector and the surroundings at the time of collection.

For example, Patent Document 1 describes an incubation medium for a solid-phase immunoassay containing lactoferrin.

### Prior Art Documents

### Patent Documents

Patent Document 1: JP1988-135865A (JP-S63-135865A)

### Summary of Invention

### Object to be solved by the invention

An object of the present invention is to provide a reagent capable of reducing a non-specific reaction in the detection of a substance to be measured in a saliva sample using a substance that is labeled with a peroxidase and that binds to the substance to be measured, a kit including the reagent, and a method for measuring a substance to be measured using the reagent.

### Means for solving the object

Examples of representative embodiments according to the present invention are described below.
<1> A reagent comprising: a glycoprotein, in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.
<2> The reagent according to <1>, in which the glycoprotein is lactoferrin or transferrin.
<3> The reagent according to <1>, in which the glycoprotein is lactoferrin.
<4> The reagent according to any one of <1> to <3>, in which the substance to be measured is a virus antigen.
<5> The reagent according to any one of <1> to <4>, in which the Substance 1 is an antibody.
<6> The reagent according to any one of <1> to <5>, further containing a membrane protein solubilizing agent.
<7> The reagent according to any one of <1> to <6>, in which a content of the glycoprotein is 0.05% by mass or more with respect to a total mass of the reagent.
<8> A kit for detecting a substance to be measured in a saliva sample, comprising: the reagent according to any one of <1> to <7>; and Substance 1.
<9> A method for detecting a substance to be measured, comprising: bringing a saliva sample into contact with the reagent according to any one of <1> to <7> to obtain a sample; and detecting the substance to be measured in the sample using Substance 1 that binds to the substance to be measured.
<10> A reagent comprising: an iron-binding protein, in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.
<11> The reagent according to <10>, in which the iron-binding protein is lactoferrin, transferrin, or ferritin.

### Effect of the invention

According to the present invention, there are provided a reagent capable of reducing a non-specific reaction in the detection of a substance to be measured in a saliva sample using substance that is labeled with a peroxidase and that binds to the substance to be measured, a kit including the reagent, and a method for measuring a substance to be measured using the reagent.

### BRIEF DESCRIPTION OF THE DRAWINGS

[Fig.1] FIG. 1 is a graph showing a relationship between a lactoferrin concentration in a sample extraction solution and a measured value measured in Example 1.
[Fig.2] FIG. 2 is a graph showing a distribution of measured values measured in Example 2.

### Embodiments for carrying out the invention

Hereinafter, the main embodiments according to the present invention will be described. However, the present invention is not limited to the specified embodiments.

In the present specification, a numerical value range described by using "to" means a range including numerical values described before and after the preposition "to" as a lower limit value and an upper limit value, respectively.

In the present specification, the total solid content refers to the total mass of components excluding a solvent from the entire components of the composition. In addition, in the present specification, the concentration of solid contents is a mass percentage of other components excluding a solvent with respect to the total mass of the composition.

In the present specification, the unit M indicates mol/L. Similarly, mM indicates mmol/L, and µM indicates µmol/L.

In the present specification, the unit N refers to a value obtained by multiplying the molar concentration by the valence of an acid or an alkali.

In the present specification, unless otherwise specified, the temperature is 23°C, the atmospheric pressure is 101,325 Pa (1 atm), and the relative humidity is 50%RH.

In addition, in the present specification, a combination of preferred aspects is a more preferred aspect.

### (Reagent)

The reagent according to the first aspect of the present invention (hereinafter, also simply referred to as "first reagent") is a reagent containing a glycoprotein, in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.

The reagent according to the second aspect of the present invention (hereinafter, also simply referred to as "second reagent") is a reagent containing an iron-binding protein, in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.

Hereinafter, in a case of being simply described as "the reagent according to the embodiment of the present invention", it shall refer to both the first reagent and the second reagent.

In addition, hereinafter, in a case of being simply described as the "specific protein", it shall refer to both the glycoprotein contained in the first reagent and the iron-binding protein contained in the second reagent.

According to the reagent according to the embodiment of the present invention, a non-specific reaction can be reduced in detection of a substance to be measured in a saliva sample using Substance 1 that is labeled with peroxidase and binds to the substance to be measured.

The reason why the above-described effect is obtained is not clear, but presumed as follows.

Since the saliva sample contains a peroxidase (POD)-like substance, it has been revealed that a non-specific reaction occurs in a case where the above-described Substance 1 is used for the detection of the saliva sample. Such a non-specific reaction is not recognized in a sample derived from a nasopharynx swab specimen or a nasal swab fluid, and is considered to be specific to a saliva sample.

Here, in the present invention, the reagent containing a glycoprotein is brought into contact with the saliva sample in the first reagent, and the iron-binding protein is brought into contact with the saliva sample in the second reagent, before the Substance 1 is brought into contact with the saliva sample.

Accordingly, it is considered that the POD-like substance is inactivated by chelating iron in the POD-like substance contained in the saliva sample, and the reaction of the POD derived from the sample is suppressed. In addition, since it is possible to remove the specific protein by washing or the like in a case of reacting with the Substance 1 by being brought into contact with the saliva sample prior to the Substance 1, it is considered that the reaction of the POD labeling the Substance 1 is less likely to be suppressed than the reaction of the POD-like substance derived from the sample.

Hereinafter, each component contained in the reagent of the present invention will be described in detail.

### <Glycoprotein>

The first reagent contains a glycoprotein.

The glycoprotein is not particularly limited, and a known glycoprotein can be used.

The glycoprotein is preferably a protein containing at least a sugar chain as a composite component.

The sugar chain moiety of the glycoprotein may be an N-bonded type or an O-bonded type. In addition, the sugar chain moiety may have a natural structure or may be artificially modified. Furthermore, the sugar chain moiety may be a neutral sugar chain or an acidic sugar chain. In addition, the sugar chain binding site in the glycoprotein may be the same site as that in the natural product, or may be a site to which the sugar chain is not bound in the natural product.

The glycoprotein is preferably a iron-binding protein.

The iron saturation of the glycoprotein contained in the reagent of the present invention may be determined in consideration of the type, stability, and the like of the glycoprotein, but for example, it is preferably set to 30% or less, more preferably set to 20% or less, and still more preferably set to 10% or less. The iron saturation may be 0%.

Among these, as the glycoprotein in the first reagent, lactoferrin or transferrin is preferable, and lactoferrin is more preferable.

In addition, these lactoferrin or transferrin may be a mutant. The mutant may include, for example, a mutant having an amino acid substitution, deletion, and/or insertion (for example, of 1 to 10 amino acids) with respect to a natural protein.

The mutant is preferably a mutant having iron binding properties.

### [Lactoferrin]

Lactoferrin is a glycoprotein having a molecular weight of approximately 80,000, which is separated from milk.

The lactoferrin is not particularly limited, and examples thereof include lactoferrin derived from mammals such as humans and cows. Examples of the lactoferrin include commercially available lactoferrin and lactoferrin prepared using genetic engineering techniques. Examples of the commercially available lactoferrin include Lactoferrin, from Bovine Milk (FUJIFILM Wako Pure Chemical Corporation), lactoferrin from human milk powder (Sigma-Aldrich), and the like, but the lactoferrin is not limited thereto.

Lactoferrin includes iron-unsaturated type (apo type) lactoferrin and iron-binding type (holo type) lactoferrin, but iron-unsaturated type (apo type) lactoferrin is preferable. In addition, these mixtures may be used. The mixing ratio can be set to a range in which the iron saturation of lactoferrin is within the above-described range.

Here, the apo type lactoferrin can also be prepared, for example, by dialysis of lactoferrin with respect to a chelating agent such as EDTA or a citric acid solution.

### [Transferrin]

Transferrin is a glycoprotein having a molecular weight of approximately 80,000, which is synthesized in the liver and the like and is contained in blood plasma.

The transferrin is not particularly limited, and examples thereof include transferrins derived from mammals such as humans, mice, and rabbits. Examples of the transferrin include commercially available lactoferrin and lactoferrin prepared using genetic engineering techniques. Examples of the commercially available transferrin include transferrin (Apo), from human blood (FUJIFILM Wako Pure Chemical Corporation), transferrin (Sigma-Aldrich), and the like, but the transferrin is not limited thereto.

Transferrin includes iron-unsaturated type (apo type) transferrin and iron-binding type (holo type) transferrin, but iron-unsaturated type (apo type) transferrin is preferable. In addition, these mixtures may be used. The mixing ratio can be set to a range in which the iron saturation of transferrin is within the above-described range.

Here, the apo type transferrin can also be prepared, for example, by dialysis of transferrin with respect to a chelating agent such as EDTA or a citric acid solution.

### [Content]

The content of the glycoprotein with respect to the total mass of the first reagent is preferably 0.05% by mass or more, more preferably 0.10% by mass or more, and still more preferably 0.20% by mass or more.

The upper limit of the content is not particularly limited, and for example, it can be set to 5% by mass or less or 2% by mass or less.

### <Iron-binding protein>

The second reagent contains an iron-binding protein.

The iron-binding protein is not particularly limited, and a known iron-binding protein can be used.

Examples of the iron-binding protein include a protein that binds to at least one of divalent iron or trivalent iron, and for example, a protein that binds to trivalent iron is also preferable.

The iron-binding protein may be any protein as long as it is a protein that binds to at least iron, and may be a protein that further binds to copper, aluminum, vanadium, zinc, gallium, or the like, in addition to iron.

The preferred range of the iron saturation in the iron-binding protein is the same as the preferred aspect of the iron saturation in the glycoprotein described above.

Among these, as the iron-binding protein in the first reagent, lactoferrin, transferrin, or ferritin is preferable, lactoferrin or transferrin is more preferable, and lactoferrin is still more preferable.

In addition, these lactoferrin, transferrin, or ferritin may be a mutant. The mutant may include, for example, a mutant having an amino acid substitution, deletion, and/or insertion (for example, of 1 to 10 amino acids) with respect to a natural protein.

The mutant is preferably a mutant having iron binding properties.

### [Lactoferrin and transferrin]

The preferred aspects of the lactoferrin and the transferrin in the iron-binding protein are the same as the preferred aspects of the lactoferrin and the transferrin in the glycoprotein described above.

### [Ferritin]

Ferritin is a soluble protein having a molecular weight of approximately 440,000, which has a hollow portion for storing iron inside.

The ferritin is not particularly limited, and examples thereof include those derived from mammals such as humans and horses. Examples of the ferritin include commercially available lactoferrin and lactoferrin prepared using genetic engineering techniques. Examples of the commercially available ferritin include apoferritin (FUJIFILM Wako Pure Chemical Corporation), ferritin from equine spleen (Sigma-Aldrich), and the like, but the ferritin is not limited thereto.

Ferritin includes iron-unsaturated type (apo type) transferrin and iron-binding type (holo type) ferritin, but iron-unsaturated type (apo type) ferritin is preferable. In addition, these mixtures may be used. The mixing ratio can be set to a range in which the iron saturation of ferritin is within the above-described range.

Here, the apo type ferritin can also be prepared, for example, by dialysis of ferritin with respect to a chelating agent such as EDTA or a citric acid solution.

### [Content]

The content of the iron-binding protein with respect to the total mass of the second reagent is preferably 0.05% by mass or more, more preferably 0.10% by mass or more, and still more preferably 0.20% by mass or more.

The upper limit of the content is not particularly limited, and for example, it can be set to 5% by mass or less or 2% by mass or less.

### <Other components>

The reagent according to the embodiment of the present invention may further contain other components.

The other components are not particularly limited, and examples thereof include buffering agents, inorganic salts, inorganic acids or inorganic bases, stabilizers, other non-specific reaction inhibitors, adsorption inhibitors, and preservatives.

Examples of the buffer agents include N-(2-acetoamido)-2-aminoethanesulfonic acid (ACES), N-(2-acetoamido)iminodiacetic acid (ADA), N,N-bis(2-hydroxyethyl)-2-aminoethanesulfonic acid (BES), N,N-bis(2-hydroxyethyl)glycine (Bicine), bis(2-hydroxyethyl)iminotris(hydroxyethyl)methane (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethanesulfonic acid (CHES), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-[4-(2-hydroxyethyl)-1-piperazinyl]ethanesulfonic acid (HEPES), 2-hydroxy-3-[4-(2-hydroxyethyl)-1-piperazinyl]propanesulfonic acid (HEPPSO), 2-(N-morpholino)ethanesulfonic acid (MES), 3-(N-morpholino)propanesulfonic acid (MOPS), 2-hydroxy-3-(N-morpholino)propanesulfonic acid (MOPSO), piperazine-1,4-bis(2-ethanesulfonic acid) (PIPES), piperazine-1,4-bis(2-hydroxy-3-propanesulfonic acid) (POPSO), N-tris(hydroxymethyl)methyl-3-aminopropanesulfonic acid (TAPS), 2-hydroxy-N-tris(hydroxymethyl)methyl-3-aminopropanesulfonic acid (TAPSO), N-tris(hydroxymethyl)methyl-2-aminoethanesulfonic acid (TES), N-[tris(hydroxymethyl)methyl]glycine (Tricine), phosphates, acetates, citrates, tris(hydroxymethyl)aminomethane, and the like.

Examples of the inorganic salts include inorganic salts known in the art, such as sodium chloride, potassium chloride, calcium chloride, magnesium sulfate, sodium hydrogen carbonate, and sodium dihydrogen phosphate.

Examples of the inorganic acids or the inorganic bases include inorganic acids or inorganic bases known in the art, such as hydrochloric acid, sulfuric acid, boric acid, phosphoric acid, sodium hydroxide, and potassium hydroxide.

Examples of the stabilizers include known chelating agents, protease inhibitors, and the like.

As the other non-specific reaction inhibitors, a compound that does not correspond to the above-described specific protein and is known in the related art as a non-specific reaction suppressor can be used.

Examples thereof include milk proteins such as skim milk, egg white albumin, commercially available blocking agents (for example, BLOCK ACE (manufactured by Sumitomo Dainippon Pharma Co., Ltd.), Blockmaster^{™} PA series (manufactured by JSR Corporation), and the like), sodium heparin, RNase, and the like.

Examples of the adsorption inhibitors include a surfactant and an adsorption inhibitor of a peptide such as albumin (for example, bovine serum albumin).

Examples of the preservatives include salicylic acid, benzoic acid, and the like.

### <Solvent>

The reagent according to the embodiment of the present invention preferably contains a solvent.

Examples of the solvent include water, a water-soluble organic solvent, and the like.

The content of water is preferably 50% to 99% by mass, more preferably 60% to 99% by mass, and still more preferably 70% to 99% by mass with respect to the total mass of the reagent.

Examples of the water-soluble organic solvent include alcohol and the like.

The content of the water-soluble organic solvent is preferably 0% to 10% by mass, more preferably 0% to 5% by mass, and still more preferably 0% to 2% by mass with respect to the total mass of the reagent.

In addition, an aspect in which the content of the water-soluble organic solvent is 0% to 0.1% by mass with respect to the total mass of the reagent is also one of the preferred aspects of the present invention.

### <Membrane protein solubilizing agent>

It is also preferable that the reagent according to the embodiment of the present invention contains a membrane protein solubilizing agent.

By containing the membrane protein solubilizing agent, for example, the reagent according to the embodiment of the present invention can be used as a pretreatment reagent (a sample extraction solution) described later.

The membrane protein solubilizing agent is not particularly limited, and known agents can be used without particular limitation. Examples thereof include sodium dodecyl sulfate (SDS), N,N-bis(3-D-gluconamidopropyl)cholamide (BIGCHAP), 3-[(3-cholamidopropyl)dimethylammonio]-1-propanesulfonate (CHAPS), N,N-bis(3-D-gluconamidopropyl)deoxycholamide (deoxy-BIGCHAP), 3-[(3-acetamidopropyl)dimethylammonio]-2-hydroxy-1-propanesulfonate (CHAPSO), n-decanoyl-N-methylglucamide (MEGA-10), n-nonanoyl-N-methylglucamide (MEGA-9), n-octanoyl-N-methylglucamide (MEGA-8), n-octyl-β-D-thioglucoside, n-octyl-β-D-maltoside, n-octyl-β-D-glucoside, sucrose monolaurate (SM1200), sucrose monocaprate (SM1000), sucrose monocholate, and the like.

### <Substance that binds to substance to be measured>

The reagent according to the embodiment of the present invention may contain a substance that is not labeled with a peroxidase and that binds to a substance to be measured.

Hereinafter, the substance that is not labeled with a peroxidase and that binds to a substance to be measured is also referred to as "Substance A". The Substance A is different from the Substance 1 in that the Substance A is not labeled with a peroxidase.

Examples of the Substance A include an antibody.

In a case of containing the Substance A, for example, the reagent according to the embodiment of the present invention can be used as a pre-reaction reagent described later.

It is preferable that the Substance A directly binds to the substance to be measured. In the present invention, the term "directly binding" means binding without interposing another molecule.

Here, the Substance A may be carried on a carrier such as a magnetic bead.

### <Use application>

The reagent according to the embodiment of the present invention is a reagent in which, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.

### [Substance 1]

The Substance 1 is a substance that is labeled with a peroxidase and binds to a substance to be measured.

Examples of the Substance 1 include an antibody, avidin, and streptavidin, which are labeled with a peroxidase.

Preferred examples of the antibody include an antibody that recognizes the substance to be measured. In addition, an antibody that recognizes the Substance A can also be used.

In addition, in a case where avidin or streptavidin is used, it is preferable that an antibody labeled with biotin binds to a substance to be measured described later.

The peroxidase is not particularly limited, and any peroxidase derived from any source may be used. Examples of thereof include a peroxidase derived from a plant, a peroxidase derived from a bacterium, and a peroxidase derived from a basidiomycete. Among these, from the viewpoints of purity, ease of availability, price, and the like, a peroxidase derived from horseradish, rice, or soybean is preferable, and a peroxidase derived from horseradish (HRP) is more preferable.

### [Substance to be measured]

The substance to be measured is not particularly limited as long as it is contained in the saliva sample and is detected by the measuring method using the Substance 1, but examples thereof include antigens such as virus antigens, bacterial antigens, or allergens; disease marker proteins such as inflammation markers or tumor markers; viruses, bacteria, human DNA or RNA, and the like.

Among these, the virus antigens are preferable.

The virus antigen is not particularly limited, and examples thereof include the full length or fragment of the spike protein of SARS-CoV-2, and the like.

An antibody or the like may be bound to the substance to be measured to form a complex.

Examples of the complex include a complex of the substance to be measured and the above-described Substance A, and the like.

Here, for example, the Substance 1 may be a substance that directly binds to the substance to be measured, or may be a substance that binds to the Substance A, that is, indirectly binds to the substance to be measured.

Examples thereof include an aspect in which an antibody that directly binds to the substance to be measured is used as the Substance 1, an aspect in which an antibody that directly binds to the substance to be measured is used as the Substance A and a substance (for example, a secondary antibody against the Substance 1) that directly binds to the Substance A is used as the Substance 1, an aspect in which an antibody that directly binds to the substance to be measured is used as the Substance A and a substance (for example, HRP-labeled avidin or HRP-labeled streptavidin) that directly binds to a label (for example, biotin or the like) of an antibody that is the Substance A is used as the Substance 1, and the like.

In addition, the method for detecting a substance to be measured in the use application of the reagent according to the embodiment of the present invention can be the same method as the method for detecting a substance to be measured according to the embodiment of the present invention.

Hereinafter, the method for detecting a substance to be measured according to the embodiment of the present invention will be described.

### (Method for detecting substance to be measured)

The method for detecting a substance to be measured according to the embodiment of the present invention includes bringing a saliva sample into contact with the reagent according to the embodiment of the present invention to obtain a sample, and detecting the substance to be measured in the sample using Substance 1 that binds to the substance to be measured.

The method for detecting the substance to be measured is not particularly limited, but can be performed, for example, by an immunological measurement method.

The immunological measurement method may be any method as long as it is a method of performing an antigen-antibody reaction using an anti-endocrine protein antibody. The measurement principle of the immunological measurement method is not particularly limited, and examples thereof include a sandwich method, a competition method, an immunochromatography method, a capillary electrophoresis method, a Western blot method, a surface plasmon resonance method (SPR method), and the like.

The measuring method in the immunological measurement method is not particularly limited, and examples thereof include an enzyme-linked immunosorbent assay (ELISA), a chemiluminescent enzyme immunoassay (CLEIA), and the like.

### <Saliva sample>

The saliva sample may be any of saliva collected from a subject or a sample obtained by subjecting the saliva to pretreatment.

### [Pretreatment]

Examples of the pretreatment include a treatment for bringing saliva into a state suitable for examination, transportation, storage, or the like, and examples thereof include pH adjustment; addition of a buffering agent, an inorganic salt, a stabilizer, a non-specific reaction inhibitor, an adsorption inhibitor, a preservative, a membrane protein solubilizing agent, or the like; and the like.

The pretreatment can be performed by bringing saliva into contact with a pretreatment reagent.

That is, the method for detecting a substance to be measured according to the embodiment of the present invention may include bringing saliva into contact with a pretreatment reagent to obtain a saliva sample (pretreatment step).

As the pretreatment reagent, the reagent according to the embodiment of the present invention can also be used.

In addition, as a pretreatment reagent that does not correspond to the reagent according to the embodiment of the present invention, a reagent that contains a buffer agent, an inorganic salt, a stabilizer, a non-specific reaction inhibitor, an adsorption inhibitor, a preservative, a membrane protein solubilizing agent, or the like, and does not contain the specific protein may be used. Preferred aspects of the buffer agent, the inorganic salt, the stabilizer, the other non-specific reaction inhibitors, the adsorption inhibitor, the preservative, and the membrane protein solubilizing agent, which are contained in such a pretreatment reagent, are the same as the preferred aspects of these components contained in the above-described reagent according to the embodiment of the present invention.

In addition, as the pretreatment reagent, a sample extraction solution known in the art can be used.

### <Pre-reaction>

The saliva sample according to the present invention may be subjected to a pre-reaction.

For example, the substance to be measured contained in the saliva sample may be brought into contact with the above-described Substance A to form a complex.

That is, the method for detecting a substance to be measured according to the embodiment of the present invention may include bringing the saliva sample into contact with the Substance A to obtain a complex of the substance to be measured and the Substance A (pre-reaction step).

The contact between the saliva sample and the Substance A may be performed by directly adding the Substance A to the saliva sample, but it is preferable to bring the saliva sample into contact with a pre-reaction reagent containing the Substance A.

As the pre-reaction reagent, the reagent according to the embodiment of the present invention can be used.

In addition, as the pre-reaction reagent, a reagent containing the substance A, a buffer agent, an inorganic salt, a stabilizer, a non-specific reaction inhibitor, an adsorption inhibitor, and the like and not containing the specific protein may be used. Preferred aspects of the Substance A, the buffer agent, the inorganic salt, the stabilizer, the other non-specific reaction inhibitors, and the adsorption inhibitor, which are contained in such a pre-reaction reagent, are the same as the preferred aspects of these components contained in the above-described reagent according to the embodiment of the present invention.

In addition, as the pre-reaction reagent, a reagent for an antibody reaction known in the art can be used.

In addition, in the pre-reaction, an antibody that directly binds to the Substance A and has a structure binding to the Substance 1 may be further reacted. Examples of such an aspect include an aspect in which the Substance A is a primary antibody, a secondary antibody labeled with biotin is further reacted with the primary antibody, and then avidin labeled with HRP or streptavidin labeled with HRP is used as the Substance 1, and the like.

### <Contact with reagent according to embodiment of present invention>

The saliva sample according to the present invention may be brought into contact with the reagent according to the embodiment of the present invention at a time point different from the above-described pretreatment and pre-reaction.

Specific examples thereof include an aspect in which the reagent according to the embodiment of the present invention is brought into contact with the saliva sample before the pretreatment, an aspect in which the reagent according to the embodiment of the present invention is brought into contact with the saliva sample after the pretreatment and before the pre-reaction, and an aspect in which the reagent according to the embodiment of the present invention is brought into contact with the saliva sample after the pre-reaction.

For example, the reagent according to the embodiment of the present invention may be added to the saliva sample at each of the above-described timings.

### <Detection of substance to be measured>

The detection of the substance to be measured is performed using the Substance 1.

The Substance 1 binds to a substance to be measured or a complex in a case where the above-described pre-reaction is performed.

Since the Substance 1 is labeled with peroxidase, the Substance 1 may be detected by a known detection method for peroxidase.

Specific examples thereof include a luminescence detection method using hydrogen peroxide and a known luminescent reagent, and an absorbance detection method using hydrogen peroxide and a known chromogenic reagent.

An example of the method for detecting a substance to be measured according to the embodiment of the present invention will be described below.

### (1) Preparation of reagent

A reagent (pretreatment reagent) according to the embodiment of the present invention, a reagent (first reagent) containing Substance A carried on particles, a reaction buffer solution (second reagent), a reagent (third reagent) containing Substance 1, a luminescent reagent (fourth reagent), a hydrogen peroxide solution (fifth reagent), and a washing solution are prepared.

The pretreatment reagent, which is a reagent according to the embodiment of the present invention, is as described above.

The first reagent is a reagent containing Substance A carried on particles. The Substance A may be carried on the particles by a known method.

As the particles, magnetic particles are preferably used.

The first reagent may contain a dispersion medium such as water, or may be used in a state of particles without containing a dispersion medium.

That is, the first reagent may be a single Substance A carried on the particles or may be in a state of further containing a dispersion medium.

The second reagent is a reaction buffer solution for performing a reaction between the Substance A in the first reagent and the saliva sample. The second reagent preferably contains a buffer agent. Examples of the buffer agent include the same compounds as the buffer agents contained in the above-described reagent according to the embodiment of the present invention, and the same applies to the preferred aspect thereof.

The third reagent is a reagent containing the Substance 1. The third reagent preferably contains the Substance 1, a buffer agent, an inorganic salt, and the like. The preferred aspects of these components are the same as the preferred aspects of these components contained in the above-described reagent according to the embodiment of the present invention.

The fourth reagent is a reagent containing a luminescent substance. Examples of the luminescent substance include known luminescent substances in the measurement of POD activity, such as luciferin and L-012 (manufactured by FUJIFILM Wako Pure Chemical Corporation).

The fourth reagent preferably further contains a buffer agent or the like. Examples of the buffer agent include the same compounds as the buffer agents contained in the above-described reagent according to the embodiment of the present invention, and the same applies to the preferred aspect thereof.

The fifth reagent is a reagent containing hydrogen peroxide.

The fifth reagent preferably further contains a buffer agent or the like. Examples of the buffer agent include the same compounds as the buffer agents contained in the above-described reagent according to the embodiment of the present invention, and the same applies to the preferred aspect thereof.

As the washing solution, a known washing solution such as water or a buffer solution can be used. In addition, a commercially available washing solution such as an Accuraseed B/F separation solution (manufactured by FUJIFILM Wako Pure Chemical Corporation) may be used.

### (2) Detection Method

First, a saliva sample is obtained by mixing saliva with a pretreatment reagent. Next, the saliva sample are mixed with the first reagent and the second reagent to react the Substance A with the substance to be measured, thereby forming a complex (Complex A). The above reaction may be performed under the conditions in a general immunological method, but can be performed, for example, by a reaction at 37°C for 1 to 10 minutes.

Subsequently, the Complex A is acquired. For example, in a case where the particles in the first reagent are magnetic particles, the Complex A can be obtained as a complex of the particles, the Substance A bonded to the particles, and the substance to be measured by collecting magnetic particles using a magnet.

Here, by washing the Complex A with a washing solution, it is possible to remove components derived from the saliva sample, components derived from the pretreatment reagent, and components derived from the second reagent, which are not bound to the particles. In particular, unnecessary specific proteins can also be removed by this washing.

Subsequently, by bringing the third reagent into contact with the complex, the Substance 1 is reacted with the Complex A to obtain Complex B, which is a complex of the Substance 1, the Substance A, the substance to be measured, and the particles. The above reaction may be performed under the conditions in a general immunological method, but can be performed, for example, by a reaction at 37°C for 1 to 10 minutes.

Here, the components in the third reagent can be removed by washing the Complex B with a washing solution. In particular, the Substance 1 in the third reagent, which is not bound to the complex, can also be removed by this washing.

Thereafter, by mixing the complex B with the fourth reagent and the fifth reagent, the substance to be measured can be detected by luminescence measurement.

In addition, as another example of the method for detecting a substance to be measured according to the embodiment of the present invention,
the measurement can also be performed in the same manner as described above using a pretreatment reagent, a reagent (first reagent) containing Substance A carried on particles, a reaction buffer solution (second reagent) which is the reagent according to the embodiment of the present invention, a reagent (third reagent) containing Substance 1, a luminescent reagent (fourth reagent), a hydrogen peroxide solution (fifth reagent), and a washing solution.

Furthermore, as another example of the method for detecting a substance to be measured according to the embodiment of the present invention, the measurement can also be performed in the same manner as described above using a pretreatment reagent, a reagent (first reagent) containing Substance A carried on particles, which is the reagent according to the embodiment of the present invention, a reaction buffer solution (second reagent), a reagent (third reagent) containing Substance 1, a luminescent reagent (fourth reagent), a hydrogen peroxide solution (fifth reagent), and a washing solution.

Even in these cases, the basic measurement method is the same.

In addition, at least one of the pretreatment reagent, the first reagent, or the second reagent may be the reagent according to the embodiment of the present invention, and an aspect in which two or more of the above-described reagents are the reagent according to the embodiment of the present invention can also be adopted.

### (Kit)

A kit according to the embodiment of the present invention is a kit for detecting a substance to be measured in a saliva sample, which contains the reagent according to the embodiment of the present invention and the Substance 1.

The kit according to the embodiment of the present invention preferably includes the reagent according to the embodiment of the present invention and the above-described third reagent.

In addition, it is preferable that the kit according to the embodiment of the present invention includes the above-described pretreatment reagent, the above-described first reagent, the above-described second reagent, the above-described third reagent, the above-described fourth reagent, and the above-described fifth reagent, and at least one of the pretreatment reagent, the first reagent, or the second reagent is the reagent according to the embodiment of the present invention.

The kit according to the embodiment of the present invention may further include the above-described washing solution.

In addition, the kit according to the embodiment of the present invention may further include a standard sample. The standard sample is preferably a sample containing the substance to be measured at a known concentration.

The standard sample is used as a calibrator, a control, or the like for creating a calibration curve showing a correlation between a known amount (concentration) of a substance to be measured contained in the standard sample and a measured value obtained by various analytical methods, confirming the effectiveness of accuracy control or calibration of an analytical instrument, and investigating the stability of quantitative analysis over time.

Examples of the above-described measured value include an absorbance, an amount of change in absorbance, a transmitted light intensity, an amount of change in transmitted light intensity, a luminescence intensity, an amount of change in luminescence intensity, and the like.

The reagent kit according to the embodiment of the present invention can further include a user manual and the like. The user manual can describe the components of the reagent according to the embodiment of the present invention, the operation procedure, the principle, and the like of the method for detecting a substance to be measured according to the embodiment of the present invention.

### Examples

Hereinafter, the present invention will be described in detail using examples. Materials, using amounts, proportions, treatment details, treatment content, and the like shown in the following examples can be appropriately changed without departing from the gist of the present invention. Accordingly, the scope of the present invention is not limited to the following specific examples. Unless otherwise specified, "parts" and "%" are based on mass.

### Example 1: Investigation of lactoferrin concentration in measurement using saliva sample

A saliva sample self-collected by a healthy subject (a person who does not exhibit cough or fever and has not been diagnosed with SARS-CoV-2 infection within the past week) was used to verify the substance having an effect of suppressing a false positive.

### 1-1 Preparation of reagent

A constitutional reagent required for the measurement was prepared using the following reagent raw materials. HEPES (2-[4-(2-hydroxyethyl)-1-piperazinyl]ethanesulfonic acid, manufactured by Dojindo Chemical Co., Ltd.), lactoferrin (manufactured by FUJIFILM Wako Pure Chemical Corporation), MES (2-morpholinoethanesulfonic acid, manufactured by Dojindo Chemical Co., Ltd.), MOPS (3-morpholinopropane-1-sulfonic acid, manufactured by Dojindo Chemical Co., Ltd.), BSA (manufactured by Sigma-Aldrich Japan Co., Ltd.), boric acid (manufactured by FUJIFILM Wako Pure Chemical Corporation), L-012 (manufactured by FUJIFILM Wako Pure Chemical Corporation), phosphoric acid (manufactured by FUJIFILM Wako Pure Chemical Corporation), and hydrogen peroxide (manufactured by FUJIFILM Wako Pure Chemical Corporation)

### (1) Sample extraction solution

Saliva collected from a healthy subject was mixed with an equal amount of a sample extraction solution consisting of the following composition to prepare a measurement sample.
"Sample extraction solution": 50 mM HEPES, and 0%, 0.1%, 0.25%, 0.5%, 0.75%, or 1.0% lactoferrin

### (2) First reagent (antigen-immobilized magnetic particle reagent)

Magnetic silica particles (trade name: Magrapid, manufactured by Sanyo Chemical Industries, Ltd.) were sequentially reacted with 3-aminopropyltriethoxysilane (manufactured by Tokyo Chemical Industry Co., Ltd.) and succinic anhydride (manufactured by FUJIFILM Wako Pure Chemical Corporation), and then magnetic particles were collected with a neodymium magnet and the supernatant was removed to obtain magnetic particles having a carboxyl group. Subsequently, using N-hydroxysuccinimide (manufactured by FUJIFILM Wako Pure Chemical Corporation) and WSC (water-soluble carbodiimide, manufactured by Dojindo Chemical Co., Ltd.), the anti-SARS-CoV-2 monoclonal antibody was reacted at 25°C for 12 to 16 hours. The magnetic particles were collected with a neodymium magnet and the supernatant was removed to prepare antibody-immobilized magnetic particles, and a first reagent consisting of the following composition was prepared.
"First reagent": 0.50 mg/mL of anti-SARS-CoV-2 monoclonal antibody-immobilized magnetic particles, 50 mM MES (pH 5.5), and 500 mM sodium chloride

### (3) Second reagent (reaction buffer solution)

A buffer solution having the following composition was prepared.
"Second reagent": 50 mM MOPS (pH 7.5) and 500 mM sodium chloride

### (4) Third reagent (antibody reagent for detection)

As a third reagent constituting a reagent for measuring the amount of antigen in the sample, a POD-labeled anti-SARS-CoV-2 monoclonal antibody was prepared by the following method.

As an anti-SARS-CoV-2 monoclonal antibody, commercially available an anti-SARS-CoV-2 antibody was digested with pepsin, and then F(ab')₂ was separated using a column (diameter: 1.5 cm × length: approximately 95 cm) filled with Sephacryl S-200HR (manufactured by Cytiva). The obtained F(ab')₂ was reduced with cysteamine hydrochloride (manufactured by Sigma-Aldrich Japan Co., Ltd.), and then Fab' was separated using a column (diameter: 1.5 cm × length: approximately 40 cm) filled with G-25 Superfine (manufactured by Cytiva). On the other hand, POD was maleimidated with a maleimidation reagent Sulfo-KMUS (manufactured by Dojindo Chemical Co., Ltd.), and the reaction mixture was subjected to a Sephadex G-25 column to remove unreacted Sulfo-KMUS, thereby obtaining maleimidated POD. The prepared Fab' was mixed with the maleimidated POD, and the mixture was separated using a Sephacryl S-100HR column to prepare a POD-labeled anti-anti-SARS-CoV-2 monoclonal antibody.

Using this, a third reagent having the following composition was prepared.
"Third reagent": 50 mM MES (pH 6.5), 20 mM enzyme-labeled anti-SARS-CoV-2 monoclonal antibody, 150 mM sodium chloride, and 2.0% BSA

### (5) Fourth reagent

A fourth reagent consisting of the following composition was prepared.
"Fourth reagent": 50 mM TAPSO, 0.9% boric acid, 0.50 mM L-012 (manufactured by FUJIFILM Wako Pure Chemical Corporation), and 200 mM thiazole phenol

### (6) Fifth reagent

A fifth reagent consisting of the following composition was prepared.
"Fifth reagent": 0.0068% phosphoric acid, and 0.0201% hydrogen peroxide (30%)

### (7) Other reagents

An Accuraseed B/F separation solution (manufactured by FUJIFILM Wako Pure Chemical Corporation) was used as a washing solution. As a sample for a calibration curve, diluted samples of a recombinant antigen (manufactured by Acro Biosystems) containing a nucleocapsid protein of SARS-CoV-2 were prepared at 1 pg/mL, 5 pg/mL, 10 pg/mL, 50 pg/mL, 100 pg/mL, 250 pg/mL, 500 pg/mL, 1,000 pg/mL, 2,500 pg/mL, and 5,000 pg/mL.

### 1-2 Measurement of SARS-CoV-2 antigen concentration

Using each of the first reagent to the fifth reagent and the washing solution, the SARS-CoV-2 antigen concentration in human saliva was measured by the following procedure using an automated chemiluminescent enzyme immunoassay analyzer Accuraseed (registered trademark).

50 µL of the first reagent was added to a reaction cuvette, followed by collecting magnetic particles using a neodymium magnet, the supernatant was removed, 50 µL of the second reagent and 25 µL of the measurement sample (saliva of a healthy subject or a calibration curve sample) were added thereto, and the mixture was stirred and heated at 37°C for 3 minutes. After completion of the heating, the magnetic particles were collected using a neodymium magnet, a reagent other than the magnetic particles was removed, and the magnetic particles were washed three times with a washing solution. Subsequently, 50 µL of a third reagent was added thereto, the mixture was heated at 37°C for 3 minutes. After completion of the heating, the magnetic particles were collected using a neodymium magnet, a reagent other than the magnetic particles was removed, and then the magnetic particles were washed three times with a washing solution. After completion of the washing, 100 µL of a fourth reagent and 100 µL of a fifth reagent were added, reacted at 37°C for 20 seconds, and then the luminescence intensity was measured. A calibration curve showing a relationship between the amount of SARS-CoV-2 antigen and the luminescence intensity from the luminescence intensity obtained by measuring each calibration curve sample, was created, and the amount of SARS-CoV-2 antigen in saliva was determined.

### 1-3 Result

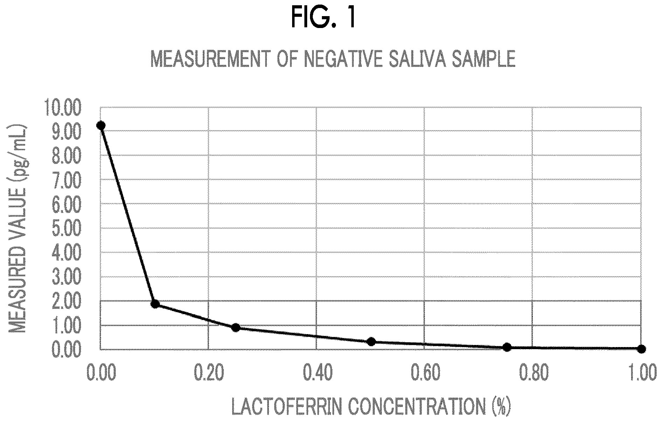

FIG. 1 shows a relationship between a lactoferrin concentration in a sample extraction solution and a measured value of an amount of SARS-CoV-2 antigen in a saliva sample derived from a healthy subject.

It was found that the measured value was decreased in a concentration-dependent manner of lactoferrin and the non-specific reaction was suppressed, as compared with a case of 0% of lactoferrin. In addition, it was confirmed that the false positive was suppressed particularly at a lactoferrin concentration of 0.5% or more.

### Example 2: Comparison of distribution of measured values using saliva sample

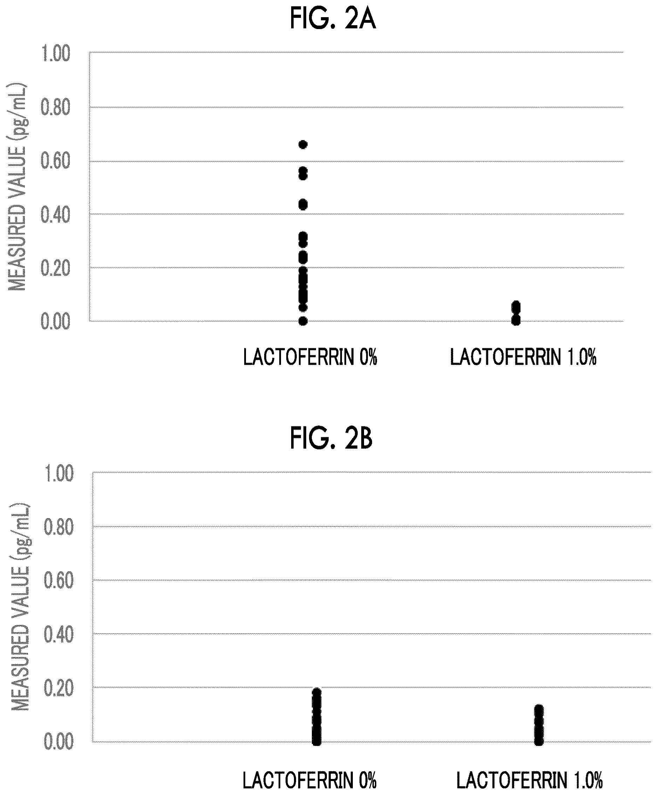

The measurement of the SARS-CoV-2 antigen in saliva samples (29 samples) self-collected by healthy subjects was performed by the same method and the same reagents as in Example 1, except that a saliva sample having the following composition was used as the sample extraction solution. FIG. 2 shows the distribution of the measured values of the amount of the SARS-CoV-2 antigen in the obtained saliva sample derived from a healthy subject.
"Sample extraction solution": 50 mM HEPES, and 0% or 1.0% lactoferrin

### Reference Example 1: Comparison of distribution of measured values using nasopharyngeal swab fluid of healthy person

The amount of SARS-CoV-2 antigen was measured by the same method as in Example 2, except that nasopharyngeal swab fluid samples (30 samples) collected from healthy subjects were used instead of the saliva sample. FIG. 2 shows the distribution of the measured values of the amount of the SARS-CoV-2 antigen in the obtained saliva sample derived from a healthy subject.

FIG. 2A is a distribution of measured values in a case where saliva samples of healthy subjects are measured in Example 2, and FIG. 2B is a distribution of measured values in a case where nasopharyngeal swab fluid samples of healthy subjects are measured in Reference Example 1. From FIG. 2A, it was confirmed that in the measurement of the saliva sample, the distribution of the measured values was converged in a case where the lactoferrin concentration in the sample extraction solution was 1.0% as compared with a case where the lactoferrin concentration in the sample extraction solution was 0%.

On the other hand, from FIG. 2B, the variation in the measured value of the nasopharyngeal swab fluid of the healthy subjects was slight even in a case where the lactoferrin concentration in the sample extraction solution was 0% as compared with the variation in the measured value of the saliva sample in FIG. 2A. In addition, it was confirmed that there was no variation and difference in a case where the lactoferrin concentration in the sample extraction solution was 1.0%.

## Claims

1. A reagent comprising:
a glycoprotein,
wherein, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.

2. The reagent according to claim 1,
wherein the glycoprotein is lactoferrin or transferrin.

3. The reagent according to claim 1,
wherein the glycoprotein is lactoferrin.

4. The reagent according to claim 1,
wherein the substance to be measured is a virus antigen.

5. The reagent according to claim 1,
wherein the Substance 1 is an antibody.

6. The reagent according to claim 1, further comprising:
a membrane protein solubilizing agent.

7. The reagent according to claim 1,
wherein a content of the glycoprotein is 0.05% by mass or more with respect to a total mass of the reagent.

8. A kit for detecting a substance to be measured in a saliva sample, comprising:
the reagent according to any one of claims 1 to 7; and
Substance 1.

9. A method for detecting a substance to be measured, comprising:
bringing a saliva sample into contact with the reagent according to any one of claims 1 to 7 to obtain a sample; and
detecting the substance to be measured in the sample using Substance 1 that binds to the substance to be measured.

10. A reagent comprising:
an iron-binding protein,
wherein, in detection of a substance to be measured in a saliva sample using Substance 1 that is labeled with peroxidase and binds to the substance to be measured, the reagent is a reagent for being brought into contact with the saliva sample prior to contact between the Substance 1 and the substance to be measured.

11. The reagent according to claim 10,
wherein the iron-binding protein is lactoferrin, transferrin, or ferritin.
